# EUROPEAN PATENT SPECIFICATION

(11) **EP 2 358 407 B1**
(45) Date of publication and mention of the grant of the patent: **09.12.2015**
(21) Application number: 09796449.8
(22) Date of filing: 15.12.2009
(51) Int. Cl.: A61L 27/44, A61L 27/34

(54) **BONE IMPLANT MATRIX AND METHOD OF PREPARING THE SAME**
KNOCHENIMPLANTATMATRIX UND HERSTELLUNGSVERFAHREN DAFÜR
MATRICE D'IMPLANT OSSEUX ET PROCÉDÉ DE PRÉPARATION DE CELLE-CI

(30) Priority: 19.12.2008 CH 19972008
(43) Date of publication of application: 24.08.2011
(73) Proprietor: Industrie Biomediche Insubri S/A, 6805 Mezzovico (CH)
(72) Inventor: PERTICI, Gianni, CH-6953 Lugaggia (CH)
(74) Representative: Postiglione, Ferruccio
(86) International application number: PCT/IB2009/007759
(87) International publication number: WO 2010/070416

(56) References cited:
- WO-A-97/46178
- WO-A-2004/098456
- WO-A-2008/088117

## Description

### Technical field of the invention

The present invention relates to implant matrixes to be used generally in the bone reconstructive surgery field, in orthopaedics and in particular in oral surgery, in dental and maxillo-facial implantology.

### State of the art

The weakness of bone structure is a well-known condition to all surgeons in the orthopaedic field, in particular in the maxillo-facial, oral and dental field. The causes are of several kinds, but quite well-known and understood. As regards means available to improve and reinforce the bones, it can be stated, that a wide variety of solutions have been proposed. Indeed, a number of techniques are currently available in the state of the art, which might be briefly distinguished in the following types: a) injections into target bone or cements or other mixtures; b) reinforcement with artificial supports to be added aside bone, being said supports metallic or polymeric o ceramic; c) use of autologous (autotransplant) human-derived, eterologous by human or animal corpse, often bovine, subject to demineralising treatments (also called Demineralized Bone Matrixes, DBM), or subject to acellularization treatments (also called Bone Graft, as for example those produced by Geistlich SA, Switzerland and RTIBiologics, USA, etc.); d) tissue engineering, an approach wherein grafts are used that are made of osteoconductive and osteoinductive materials, such as e.g. some bioglasses; f) regenerative medicine, where artificial or semi-artificial matrixes are used in order to host and deliver living cells to target area and thus enhance the formation of new resistant bone.

The introduction of new implant materials has allowed a remarkable development in bone reparative and reconstructive surgery in the last decades.

Choosing an implant material is based on the osteogenesis, osteoinduction and osteoconduction properties of the material thereof.

The most efficient solution among those currently available is to use an autologous bone as implant material, which implies, however, some disadvantages and risks.

The quantity of material available to prepare the implant is limited and moreover the patient must undergo a dual intervention, the first for removing autologous bone and the second for the subsequent implant.

According to the currently available techniques, an alternative way is represented by the usage of homologous tissue, that is the usage of demineralised bone matrix (DBM).

The DBM is obtainable by a living donor, by a donor corpse or an animal. The human cortical derived DBM, however, has the same drawback of the autologous bone, i.e. the quantity of available material appears to be reduced. Further risks exist connected to possible infections, in particular viral and to potential compatibility problems, since the material to be implanted is of a heterologous nature. Moreover, the psychological aspect for the recipient, above all when the material is corpse-derived, represents an unnegligible critical element.

As it is known in the state of the art, as an alternative the DBM may be of animal origin, in particular bovine. In the latter case, however, a microscopic examination can show that the bovine DBM porosity is higher than the cortical-derived human DBM, with a resulting minor compatibility and a reduced predisposition to the cell rooting and the growth of new integrated functional tissue.

The Applicant has observed that the higher porosity and the chemical structure of the bovine DBM result into a lower mechanical resistance, with a consequent higher weakness thereof. Such a weakness is particularly disadvantageous both in the pre-implant step, since shaping the matrix, according to the shape of the bone cavity which will host the matrix itself, with the desired accuracy is not easy, and in the implant *in situ* positioning step, because of the poor toughness of the matrix itself, which is often subject to a fragile fracture during the clamping steps.

Moreover, just because of the material weakness, the positioning and insertion of clamping elements (for example screws) in such a matrix, is difficult and not enough accurate and, as stated before, it often causes the matrix break.

Moreover, during the shaping step of the matrix currently available in the state of the art, such as, for example, demineralised bone matrixes (DBM), acellularised (bone grafts), bioglasses, bio-ceramics, etc., disadvantageously, undesirable powders are formed. For example, in dentistry, the shaping step takes place just before the implantation and the resulting powders creep up also on the matrix to be implanted.

Another proposed solution is a composite osteoimplant described in US2008/0063684. Such an osteoimplant includes a polymer and bone-derived particles. The composite is adapted and constructed to be formable during or immediately prior to implantation and to be set after final surgical placement.

US 7.270.813 describes a method for preparing bone-derived composites, wherein the mineral portion of the bone is treated with a coupling agent before being incorporated into a biocompatible polymeric matrix. The resulting composite may be used as such that or be further processed to form an osteoimplant.

WO 97/46178 A discloses a porous biomaterial, comprising hydroxyapatite matrix impregnated with gelatin or collagen or PLA monomers, which are then polymerized or cross-linked in situ coating the micropores of the matrix with the resulting polymer, in order to reinforce the mechanical properties of the resulting material.

WO 2004/098456 A discloses a biostructure to be used for bone repair, said biostructure comprising a composed base matrix including particles of demineralized bone matrix bound together with a binder such as collagen, gelatin, starch, said composed base matrix treated with an interpenetrating agent (PCL) subsequently treated, by option, with a third material, such as growth factors or antibiotics, wherein said third material is in at least some space not occupied by either the matrix or the interpenetrant.

WO 2008/088117 A discloses a composite of demineralized bone matrix (DBM)- carboxymethyl cellulose (CMC)/glycerol using a cellulose-base CMC and glycerol as an DBM stabilizer wherein the DBM is a bone powder.

Therefore, there is the need in the field of regenerative bone surgery to find new bone implant matrixes, which have satisfactory characteristics of mechanical resistance and ductility to tri-dimensional processing.

### Summar of the invention

Object of the present invention is a matrix for bone implant, comprising a base matrix treated with a reinforcing mixture as defined in the claims.

The bone implant matrix object of the present invention is suitable to be used in the bone reconstructive surgery field in general, in orthopaedics and in particular in the oral surgery, in the maxillo-facial and dental implantology.

### List of figures

Particular embodiments of the invention are described in detail herein below, as a way of example and not limited to, with reference to the attached figures, wherein:
- Figure 1 is an optical microscopy image of the cortical layer of human bone,
- Figure 2 is an optical microscope image of a bovine-derived purified bone,
- Figure 3 is an optical microscope image of a bovine-derived bone reinforced according to a preferred embodiment of the invention,
- Figure 4 is a scanning electronic microscope (SEM) image of a section of a bone implant matrix made according to a preferred embodiment of the invention which subsequently underwent a cell seeding, with chondrocyte-type cells, though the micro-seeding technique. The image was obtained on the third day after the seeding;
- Figure 5 shows graphs of the monoaxial mechanical crushing strength of a bovine-derived acellularised matrix (untreated) and an analogous matrix, treated according to an example of the invention.

### Detailed description of the invention

A bone implant matrix, according to an embodiment of the invention, comprises:
a) a base matrix treated with
b) a reinforcing mixture containing at least a polymer, as defined the claims.

By the expression "base matrix" a substantially solid tri-dimensional body is meant, intended, typically porous, after a treatment described herein below, to be implanted in bone cavities.

The base matrix may be natural. The natural base matrixes may be selected, for example, from demineralised bone, non-demineralised bone, acellularised bone, all of bovine bone origin. As it is known, the acellularised bone matrixes are non-demineralised matrixes completely (or substantially) devoid of the donor's cellular material.

The bone implant matrixes may have different shapes and dimensions, such as to be adapted according to the shape and the dimensions of the bone cavities, where said matrixes can be implanted.

For example, such bone matrixes may be parallelepiped-shaped, in particular cube-shaped.

The dimensions of the bone implant matrixes, for example, can vary from a few mm till some dm of maximum length.

Basic matrixes are usually porous.

The base matrix and the polymer (s) of the reinforcing mixture are advantageously bio-compatible.

Furthermore, the base matrix and/or the polymer of the reinforcing mixture are preferably bio-integrable, in order to better assist the growth of the new bone integrated with the surrounding tissue.

Moreover by the expression "reinforcing mixture" it is meant a mixture comprising at least a polymer, i.e. which comprises only one polymer or, alternatively, it may be multi-polymeric, i.e. may comprise more than one polymer at the same time.

In particular, by the expression reinforcing mixture it is meant a mixture, wherein the synthetic or natural, and advantageously bio-compatible, polymer or polymers, are finely dispersed.

Particularly preferred is a reinforcing mixture obtained starting from two solutions, each of them made of a soluble polymer and an agent promoting the cell engraftment, growth and proliferation, and the tissue integration, respectively, immiscible to each other, but made partially miscible by adding an alcohol or another proper solvent in each of them; in order to obtain a fine and homogeneous molecular dispersion of the components, which, during the solvent evaporation step creates a homogeneous coating, finely dispersed on the whole surface of the porous bone matrix, i.e. coating it also in the inner cavities, without closing them, though.

The polymer of the reinforcing mixture may be selected, for example, from the group comprising biodegradable polymers, polyesters are preferred, in particular polylactic acid (PLA), polyglycolic acid (PGA), polycaprolactone (PCL) and co-polymers thereof, such as for example polycaprolactone-polylactic (PLA/PCL) co-polymers, poly(L-lactide-co-ε-capcolactone) co-polymers.

Moreover the polymer may be selected from the group comprising starch, poly(caprolactones), poly(L-lactide), poly(D,L-lactide-co-glycolide), poly(L-lactide-co-D,L-lactide), their enantiomers, their co-polymers and mixtures thereof.

According to another embodiment of the invention, the reinforcing mixture may comprise, beside the polymer or the polymers, at least an additional component selected from cell nutrients, cell-growth promoters, cell-adhesion promoters, osteoinductors, os-teointegrators, "friendliness to cell".

By the expression "friendliness to cell" it is meant a substance, which is able to promote the cell-rooting and the cell growth, by stimulating cell proliferation and tissue integration.

According to a preferred embodiment of the invention the "friendliness to cell" may be selected from gelatine, in particular hydrolysed. The presence of at least one "friendliness to cell" assists the cell rooting and growth, since the cell proliferation and the tissue integration are promoted and this is an important advantage in respect to the prior art.

The solvents used to prepare the reinforcing mixture are commonly known in the state of the art and may be, for instance, dichloromethane, tetrahydrofuran, isopropanol, etc., and their specific use results from the kind of polymer/s used, according to the well-known laws of chemistry.

A particularly preferred embodiment of the bone implant matrix comprises a base matrix, which is a bovine demineralised bone matrix treated with a reinforcing mixture comprising a biodegradable polyester-based co-polymer, such as, for example, a polycaprolactone-polylactide co-polymer (PLA/PCL) and, preferably hydrolysed, gelatine.

According to a further particularly preferred embodiment, the bone implant matrix comprises a base matrix, which is a bovine non-demineralised accellularised bone matrix, treated with a reinforcing mixture comprising a biodegradable polyester-based co-polymer, such as, for example, a poly(L-lactide-co-ε-caprolactone), and preferably hydrolysed, gelatine.

In another preferred embodiment, the reinforcing mixture comprises at least a biodegradable polyester and at least a "friendliness to cell".

According to a particular embodiment, further a kit may be realized, comprising the reinforcing mixture in a proper separate container and the base matrix to be treated.

The bone implant matrixes described above may be used in the orthopedic, oral surgery, the bone reconstructive surgery and implantology.

Said bone implant matrixes are particularly suitable to be used in oral surgery, in maxillo-facial and dental bone reconstructive surgery, in particular to reconstruct and consolidate the bone structures before carrying out the insertion of dental implants.

According to a preferred usage, said bone implant matrixes are particularly suitable in the bone reconstructive surgery, following the decrease in the bone mass in patients affected by osteoporosis.

Additionally, said bone implant matrixes may be used also in oral and dental applications, dentistry, as bone "chips", as support matrixes for cell housing and in cellular therapies.

The bone implant matrixes may be used for both human and veterinary use.

A method for preparing the bone implant matrixes is be described herein below, which comprises the following steps:
a) preparing a solution of a reinforcing mixture containing at least a polymer,
b) immersing a base matrix into the reinforcing mixture made according to step a),
c) drying and degassing the matrix made according to step b), preferably in a vacuum furnace at 37°C (±2°C) for 24 hours, for removing possible solvent residues (for example in air or preferably in a vacuum furnace).

Drying and degassing the bone implant matrix usually take place contemporarily.

Such a method may optionally be followed by a post-treatment step, which comprises for example heating, conditioning in a inert atmosphere the bone implant matrixes and degassing to remove completely the possible residues of solvents used in the preparation process.

Moreover, the bone implant matrix preparation process may be followed by a packaging method which comprises the steps of:
d) packaging in a sterile and inert atmosphere,
e) sterilization (preferably through either gamma-ray or beta-ray irradiation).

The matrixes known in the state of the art and commonly used in the orthopaedic surgery have poor mechanical resistance, poor fixing resistance, fragility and little ductility characteristics.

The Applicant has surprisingly found that, by treating a base matrix with a reinforcing mixture containing at least a polymer, as described above, it is possible to obtain bone implant matrixes, which have such ductility characteristics as to make easier shaping, in the pre-implant step, the bone implant matrix, with the desired accuracy, in relation to the bone cavity, which will host the matrix itself. Moreover, the mechanical resistance of the bone implant matrixes, obtainable according to the described embodiments, determines a reduced trend to the fragile fracture of the matrixes themselves, this is particularly advantageous both in the implant *in situ* positioning step and in the positioning and insertion of clamping elements (for example screws), of such a matrix.

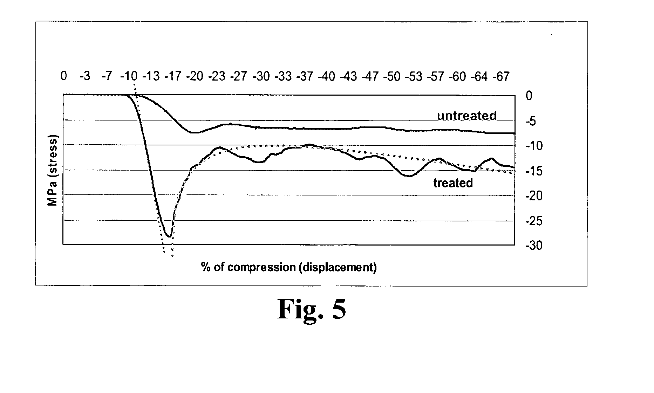

Figure 5 depicts two graphs of monoaxial mechanical crushing strength: the curve "untreated" refers to a bovine-derived acellularised matrix and the curve "treated" refers to an analogue acellularised matrix, treated with a reinforcing mixture, containing at least one polymer, as previously disclosed. The clear comparison indicates how the previously disclosed treatment allows the matrix to be stiffer (increased modulus of elasticity, or Young's modulus) and have a higher load resistance (maximum effort, in MPa).

The bone implant matrixes may be subject, during the pre-implant step, to a possible cell-seeding process.

The cells can be seeded by using seeding techniques known in the state of the art, preferably with micro-seeding technique after the matrix has been brought to a temperature of 37°C (preferably in an incubator). The seeded bone implant matrix is then covered with a proper culturing medium and kept inside the incubator according to the techniques commonly required by the cell type used.

For each centimetre of the bone implant matrix, the cell optimal load is about 300000-500000 cell/cm³.

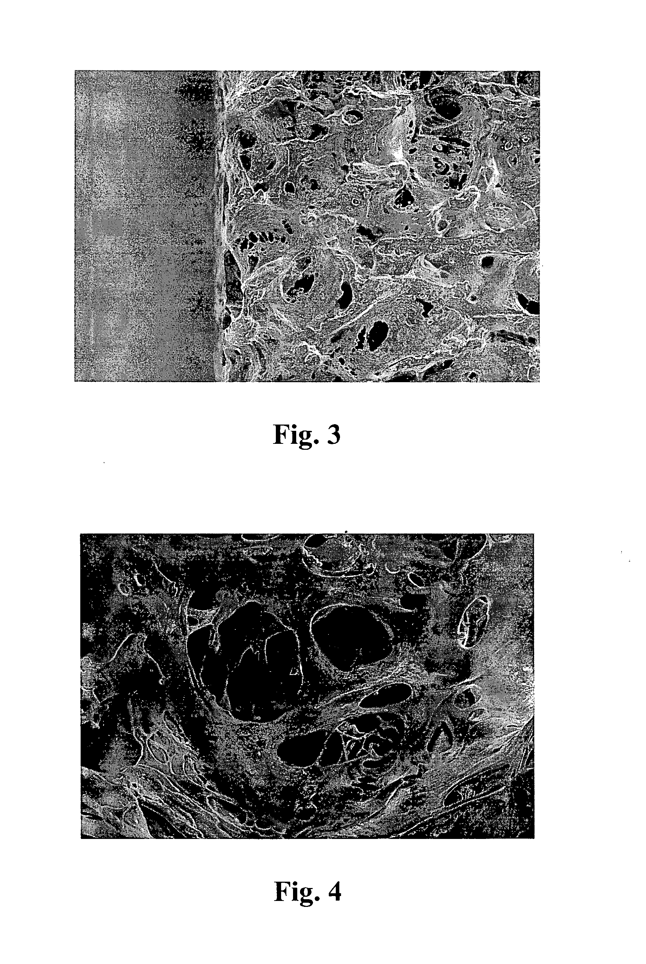

Figure 4 shows how the bone implant matrix according to an embodiment of the invention allows the cell rooting, due to the porosity characteristics, in terms of both dimensions and available spaces.

The presence of a lattice formed by the chondrocytes inside the porous spaces of the matrix is, indeed, pointed out.

The following, not limitative, examples describe embodiments of the invention.

### EMBODIMENT EXAMPLE 1. Method for preparing a bone implant matrix

Bring into solution 1 g of polymer in 20 ml of dichloromethane.

Prepare 20 ml of 1.5% solution of hydrolysed porcine gelatine.

Add 10 ml of isopropanol to the polymeric solution previously prepared.

Keep the obtained polymeric solution under stirring for 15 minutes.

Add the porcine gelatine, previously prepared, to the polymeric solution.

Keep the polymeric solution so obtained well stirred for at least 5 minutes.

Immerse the base matrix into the polymeric solution and keep immersed for at least 30 minutes.

At the end, dry the product in the air for at least 24 hours.

Optionally, treat subsequently in a furnace, to remove the remaining solvent from the bone implant matrix (T<40°C).

### EMBODIMENT EXAMPLE 2A. Method for preparing a bone implant matrix

Bring into solution 1 g PLA/PCL co-polymer in 20 ml of dichloromethane. Keep under stirring with a magnetic stirrer for at least 45 minutes at room temperature, under stirring at about 100 rpm.

Prepare 20 ml of 1.5% solution of hydrolysed porcine gelatine. Pour water, preferably by injection, and gently stirring, add the hydrolysed porcine gelatine. Keep under stirring for at least 1 hour at 37°C (± 2°C), under stirring at about 100 rpm.

Add 10 ml of isopropanol to the PLA/PCL co-polymeric solution in dichloromethane previously prepared.

Keep the so obtained polymeric solution under stirring for 20 minutes.

Add the gelatine solution, previously prepared, to the co-polymeric solution.

Keep the polymeric solution so obtained well stirred for 10 minutes at room temperature, under stirring at about 180 rpm.

Immerse the demineralised bone matrix into the polymeric solution and keep immersed for at least 30 minutes under stirring (about 200 rpm).

At the end, insert the product in the vacuum furnace for at least 24 hours at 37°C (± 2°C).

### EMBODIMENT EXAMPLE 2B. Method for preparing a bone implant matrix.

Bring into solution 1 g PLA/PCL co-polymer in 20 ml of dichloromethane. Keep under stirring for at least 45 minutes at room temperature in order to obtain a solution having a very homogeneous dispersion.

Prepare 20 ml of 1.5% solution of hydrolysed porcine gelatine. Pour water, preferably by injection, and gently stirring, add the hydrolysed porcine gelatine. Keep under stirring for at least 1 hour at 37°C (± 2°C), in order to obtain a solution characterized by a very homogeneous dispersion.

Add 10 ml of isopropanol to the PLA/PCL co-polymeric solution in dichloromethane previously prepared.

Keep the so obtained co-polymeric solution under stirring for 20 minutes.

Add the gelatine solution, previously prepared, to the co-polymeric solution.

Keep the polymeric solution so obtained well stirred for 10 minutes at room temperature, in order to obtain a stable, well homogeneous and nano-dispersed solution of all the compounds used.

Immerse the bovine non-demineralised, acellularized bone matrix into the polymeric solution and keep immersed for at least 30 minutes under stirring.

At the end, insert the product in the vacuum furnace for 24 hours at 37°C (± 2°C) in order to remove the solvents.

### EMBODIMENT EXAMPLE 2C. Method for preparing a bone implant matrix.

Bring into solution 1 g P(L,DL)LA co-polymer in 20 ml of dichloromethane. Keep under stirring for at least 45 minutes at room temperature in order to obtain a solution having a very homogeneous dispersion.

Immerse the bovine bone matrix into the polymeric solution and keep immersed for at least 30 minutes under stirring.

Insert the product in the vacuum furnace for 24 hours at 37°C (± 2°C) in order to remove the solvents.

Immerse again the matrix, previously treated in the polymeric solution and keep immersed for at least 30 minutes under stirring.

Insert the product in the vacuum furnace for 24 hours at 37°C (± 2°C) in order to remove the solvents.

Prepare 20 ml of 1.5% solution of hydrolysed porcine gelatine. Pour water, preferably by injection, and, gently stirring, add the hydrolysed porcine gelatine. Keep under stirring for at least 1 hour at 37°C (± 2°C), in order to obtain a solution characterized by a very homogeneous dispersion.

Add 10 ml of isopropanol to the P(L,DL)LA co-polymeric solution in dichloromethane, previously prepared.

Keep the so obtained co-polymeric solution under stirring for 20 minutes.

Add the gelatine solution, previously prepared, to the co-polymeric solution.

Keep the polymeric solution so obtained well stirred for 10 minutes at room temperature, in order to obtain a stable, well homogeneous and nano-dispersed solution of all the compounds used.

Immerse the already twice treated-bovine non-demineralised, acellularized bone matrix into the last polymeric solution and keep immersed for at least 30 minutes under stirring.

At the end, insert the product in the vacuum furnace for 24 hours at 37 °C (± 2°C) in order to remove the solvents.

### EXAMPLE 3. Experimental tests

The optical microscope scanning of several substrates was carried out, using the ESEM system Evo 50 EP by Zeiss-Cambridge Instruments (Germany).

All the images were obtained with the same magnitude: 30X.

Moreover, all the images were normalised at the same proportions.

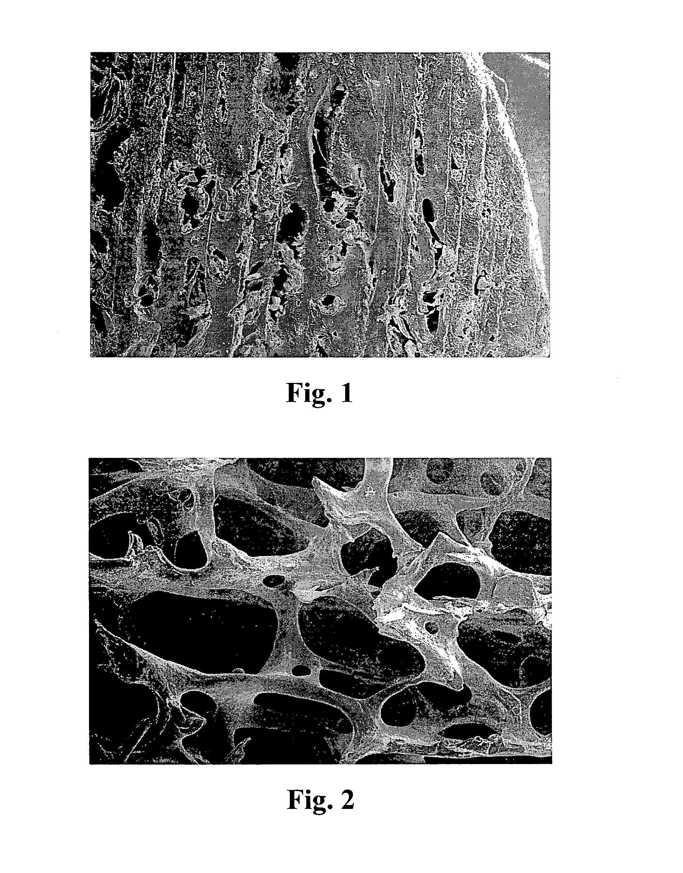

Figure 1 shows an image obtained by the optical microscope of a human bone cortical layer, which presents the desirable porosity and mechanical resistance characteristics.

Figure 2 shows an image obtained by an optical microscope of a bovine demineralised bone matrix. In the microscopic structure, remarkable differences may be highlighted in respect to the human bone cortical layer (figure 1), in particular concerning the porosity. The latter appears much higher and poorly compatible with the cell rooting.

The bovine demineralised bone matrix in figure 2, which represents a "base matrix" according to an embodiment of the invention, underwent a treatment with a reinforcing mixture containing at least a polymer, in particular containing a polycaprolactone-polylactic co-polymer (PLA/PCL) and hydrolysed gelatine.

The image of the reinforced bovine demineralised bone matrix obtained by the optical microscope is shown in figure 3.

As it can be seen by comparing figure 1 and figure 3, the microscopic structure of the bone implant matrix in figure 3 obtained according to an embodiment of the invention shows strong similarity to the human bone cortical layer. In particular, concerning the porosity, which appears completely comparable in terms of both dimensions and available spaces for the cell rooting.

The preliminary mechanical (Fig. 5) and pre-implant tests have confirmed an adequacy of the matrix shown in figure 3 in implantology for human use, the behaviour of such a matrix is particularly satisfactory, if compared to the behaviour of the human bone cortical layer.

## Claims

1. A bone implant matrix comprising:
a) a base matrix treated with
b) a reinforcing mixture as a homogeneous coating, finely dispersed on the whole surface of the matrix, said reinforcing mixture containing at least a polymer and at least an additional component selected from cell nutrients, cell-growth promoters, cell-adhesion promoters, osteo-inductors, osteo-integrators, "friendliness to cell": a substance, which is able to promote the cell-rooting and the cell growth, by stimulating cell proliferation and tissue integration, wherein the base matrix is a bone demineralised, non-demineralised, acellularized bovine matrix, the polymer of the reinforcing mixture is a biodegradable polyester or co-polymer thereof, the "friendliness to cell" is selected from the group consisting of gelatine, hydrolysed gelatine and wherein the reinforcing mixture is obtained starting from two solutions, each of them made of a soluble polymer, which is the biodegradable polyester or co-polymer thereof, and an agent, which is the gelatine or hydrolysed gelatine, promoting the cell engraftment, growth and proliferation, and the tissue integration, respectively, immiscible to each other, but made partially miscible by adding an alcohol or another proper solvent in each of them; in order to obtain a fine and homogeneous molecular dispersion of the components.

2. A bone implant matrix according to claim 1, wherein the bio-degradable polyester is selected from the group consisting of polylactic acid (PLA), poliglycolic acid (PGA), polycaprolactone (PCL) and co-polymers thereof comprising polycaprolactone-polylactic (PLA/PCL) co-polymers, poly(L-lactide-co-ε-capcolactone) co-polymers, poly(L-lactide), poly(D,L-lactide-co-glycolide), poly(L-lactide-coD,L-lactide), their enantiomers, their co-polymers and mixtures thereof.

3. A bone implant matrix according to claim 1, wherein the reinforcing mixture containing at least a polymer and at least an additional component selected from the group consisting of gelatine, hydrolysed gelatine, wherein the base matrix is a bone non-demineralised, acellularized bovine matrix and the polymer of the reinforcing mixture is a biodegradable polyester or co-polymer thereof selected from the group consisting of polylactic acid (PLA), poliglycolic acid (PGA), polycaprolactone (PCL) and co-polymers thereof comprising polycaprolactone-polylactic (PLA/PCL) co-polymers, poly(L-lactide-co-ε-capcolactone) co-polymers, poly(L-lactide), poly(D,L-lactide-co-glycolide), poly(L-lactide-coD,L-lactide), their enantiomers, their co-polymers and mixtures thereof.

4. A bone implant matrix according to claim 1, comprising a bovine demineralised bone matrix treated with a reinforcing mixture comprising a biodegradable polyester-based co-polymer and hydrolysed gelatin.

5. A bone implant matrix according to claim 1, comprisinga bovine acellularised bone matrix, treated with a reinforcing mixture comprising a biodegradable polyester-based copolymer and hydrolized gelatin.

6. A bone implant matrix according to claim 1, comprising a bovine non-demineralised acellularised bone matrix, treated with a reinforcing mixture comprising a biodegradable polyester-based copolymer and hydrolized gelatin.

7. A bone implant matrix according to claims 4-6, wherein the biodegradable polyester-based co-polymer is a polycaprolactone-polylactic copolymer (PLA/PCL).

8. A bone implant matrix according to claims 4-6, wherein the biodegradable polyester-based co-polymer is a poly(L-lactide-co-ε-caprolattone) co-polymer.

9. A bone implant matrix according to claim 1, for use in at least one of the following fields: bone reconstructive surgery, in maxillo-facial bone reconstructive surgery, in oral surgery, dental surgery, orthopaedic surgery and implantology.

10. A bone implant matrix according to claim 1, for use in human or veterinary treatment.

## Patentansprüche

1. Knochenimplantatsmatrix, die umfasst:
a) eine Basismatrix, die behandelt ist mit:
b) einer verstärkenden Mischung als homogene Beschichtung, die auf der gesamten Oberfläche der Matrix fein dispergiert ist, wobei die verstärkende Mischung zumindest ein Polymer und zumindest eine weitere Komponente enthält, die aus Zellnährstoffen, Zellwachstumspromotoren, Zelladhäsionspromotoren, Osteoinduktoren, Osteointegratoren, "Zellfreundlichkeit": eine Substanz, die in der Lage ist, die Zellverwurzelung und das Zellwachstum durch Stimulieren der Zellproliferation und der Gewebeintegration zu fördern, wobei die Basismatrix eine entmineralisierte, nicht-entmineralisierte, azellularisierte Rinderknochenmatrix ist, wobei das Polymer der verstärkenden Mischung ein biologisch abbaubares Polyester oder Copolymer davon ist, wobei die "Zellfreundlichkeit" aus der Gruppe ausgewählt ist, bestehend aus Gelatine und hydrolysierter Gelatine, und wobei die verstärkende Mischung ausgehend von zwei Lösungen erhalten wird, wobei jede davon aus einem löslichen Polymer, das das biologisch abbaubare Polyester oder Copolymer davon ist, und einem Mittel hergestellt ist, das die Gelatine oder hydrolysierte Gelatine ist, wobei das Einwachsen, das Wachstum und die Proliferation von Zellen bzw. die Gewebeintegration gefördert werden, die nicht miteinander vermischbar sind, aber durch Zugabe eines Alkohols oder eines andere geeigneten Mittels in jedem davon teilweise vermischbar gemacht werden; um eine feine und homogene molekulare Dispersion der Komponenten zu erzielen.

2. Knochenimplantatsmatrix nach Anspruch 1, wobei das biologisch abbaubare Polyester aus der Gruppe ausgewählt ist, bestehend aus Polymilchsäure (PLA), Polyglykolsäure (PGA), Polycaprolacton (PCL) und Copolymeren davon, umfassend Polycaprolacton-Polymilch-(PLA/PCL-)Copolymere, Poly(L)-lactid-co-ε-caprolacton)-Copolymere, Poly(L-lactid), Poly(D,L-lactid-co-glykolid), Poly(L-lactid-co-D,L-lactid), deren Enantiomeren, deren Copolymeren und Mischungen davon.

3. Knochenimplantatsmatrix nach Anspruch 1, wobei die verstärkende Matrix zumindest ein Polymer und zumindest eine weitere Komponente enthält, die aus der Gruppe ausgewählt ist, bestehend aus Gelatine und hydrolysierter Gelatine, wobei die Basismatrix eine nicht-entmineralisierte, azellularisierte Rinderknochenmatrix ist und das Polymer oder Copolymer davon aus der Gruppe ausgewählt ist, bestehend aus Polymilchsäure (PLA), Polyglykolsäure (PGA), Polycaprolacton (PCL) und Copolymeren davon, umfassend Polycaprolacton-Polymilch-(PLA/PCL-)Copolymere, Poly(L)-lactid-co-ε-caprolacton)-Copolymere, Poly(L-lactid), Poly(D,L-lactid-co-glykolid), Poly(L-lactid-co-D,L-lactid), deren Enantiomeren, deren Copolymeren und Mischungen davon.

4. Knochenimplantatsmatrix nach Anspruch 1, die eine entmineralisierte Rinderknochenmatrix umfasst, die mit einer verstärkenden Mischung behandelt ist, die ein biologisch abbaubares polyesterbasiertes Copolymer und hydrolysierte Gelatine umfasst.

5. Knochenimplantatsmatrix nach Anspruch 1, die eine azellularisierte Rinderknochenmatrix umfasst, die mit einer verstärkenden Mischung behandelt ist, die ein biologisch abbaubares polyesterbasiertes Copolymer und hydrolysierte Gelatine umfasst.

6. Knochenimplantatsmatrix nach Anspruch 1, die eine nicht-entmineralisierte azellularisierte Rinderknochenmatrix umfasst, die mit einer verstärkenden Mischung behandelt ist, die ein biologisch abbaubares polyesterbasiertes Copolymer und hydrolysierte Gelatine umfasst.

7. Knochenimplantatsmatrix nach den Ansprüchen 4 bis 6, wobei das biologisch abbaubare polyesterbasierte Copolymer ein Polycaprolacton-Polymilch-(PLA/PCL-)Copolymer ist.

8. Knochenimplantatsmatrix nach den Ansprüchen 4 bis 6, wobei das biologisch abbaubare polyesterbasierte Copolymer ein Poly(L)-lactid-co-ε-caprolacton)-Copolymer ist.

9. Knochenimplantatsmatrix nach Anspruch 1 zur Verwendung in zumindest einem der folgenden Gebiete: rekonstruierende Knochenchirurgie, rekonstruierender Kiefer- und Gesichtschirurgie, Mundchirurgie, Zahnchirurgie, orthopädischer Chirurgie und Implantologie.

10. Knochenimplantatsmatrix nach Anspruch 1 zur Verwendung bei der Behandlung von Mensch oder Tier.

## Revendications

1. Matrice pour implant osseux comprenant :
a) une matrice de base traitée par
b) un mélange de renfort comme revêtement homogène, finement dispersé sur toute la surface de la matrice, ledit mélange de renfort contenant au moins un polymère et au moins un composant supplémentaire choisi parmi des nutriments pour cellules, des promoteurs de croissance cellulaire, des promoteurs d'adhésion cellulaire, des ostéo-inducteurs, des ostéo-intégrateurs, un "milieu hôte pour les cellules" : une substance capable de promouvoir l'enracinement des cellules et leur croissance en stimulant la prolifération cellulaire et l'intégration tissulaire, dans laquelle la matrice de base est un matrice osseuse bovine déminéralisé, non- déminéralisé, décellularisée, le polymère du mélange de renfort est un polyester biodégradable ou un de ses copolymères, le "milieu hôte pour les cellules" est choisi dans le groupe constitué de la gélatine, la gélatine hydrolysée, et dans laquelle le mélange de renfort est obtenu en partant de deux solution, chacune constituée d'un polymère soluble qui est un polyester biodégradable ou un de ses copolymères, et un agent qui est la gélatine ou la gélatine hydrolysée, promouvant l'enracinement des cellules, leur croissance et leur prolifération, et l'intégration tissulaire, respectivement, immiscibles l'un à l'autre, mais partiellement miscibles en ajoutant un alcool ou un autre solvant adéquat dans chacun d'eux ; afin d'obtenir une dispersion moléculaire fine et homogène des composants.

2. Matrice pour implant osseux selon la revendication 1, dans laquelle le polyester biodégradable est choisi dans le groupe constitué du polyacide lactique (PLA), du polyacide glycolique (PGA), de la polycaprolactone (PCL) et de leurs copolymères, comprenant des copolymères polycaprolactone-acide polylactique (PLA/PCL), des copolymères polyacide-L-lactique-poly-c-caprolactone, le polyacide L-lactique, le copolymère polyacide-D,L-lactique-acide-glycolique, le copolymère polyacide-L-lactique-polyacide-D,L-lactique, leurs énantiomères, leurs copolymères et leurs mélanges.

3. Matrice pour implant osseux selon la revendication 1, dans laquelle le mélange de renfort contenant au moins un polymère et au moins un composant supplémentaire choisi dans le groupe constitué du groupe consistant en la gélatine et la gélatine hydrolysée, dans laquelle la matrice de base est une matrice osseuse bovine non-déminéralisée décellularisée et le polymère du mélange de renfort est un polyester biodégradable ou un de ses copolymères choisi dans le groupe constitué du polyacide lactique (PLA), du polyacide glycolique (PGA), de la polycaprolactone (PCL) et de leurs copolymères, comprenant des copolymères polycaprolactone-acide polylactique (PLA/PCL), des copolymères polyacide-L-lactique-poly-ε-caprolactone, le polyacide L-lactique, le copolymère polyacide-D,L-lactique-acide-glycolique, le copolymère polyacide-L-lactique-polyacide-D,L-lactique, leurs énantiomères, leurs copolymères et leurs mélanges.

4. Matrice pour implant osseux selon la revendication 1 comprenant une matrice osseuse bovine déminéralisée traitée par un mélange de renfort comprenant un copolymère biodégradable à base de polyester et de la gélatine hydrolysée.

5. Matrice pour implant osseux selon la revendication 1 comprenant une matrice osseuse bovine décellularisée traitée par un mélange de renfort comprenant un copolymère biodégradable à base de polyester et de la gélatine hydrolysée.

6. Matrice pour implant osseux selon la revendication 1 comprenant une matrice osseuse bovine non-déminéralisée décellularisée traitée par un mélange de renfort comprenant un copolymère biodégradable à base de polyester et de la gélatine hydrolysée.

7. Matrice pour implant osseux selon les revendications 4-6, dans laquelle le copolymère biodégradable à base de polyester est un copolymère polycaprolactone-acide polylactique (PLA/PCL).

8. Matrice pour implant osseux selon les revendications 4-6, dans laquelle le copolymère biodégradable à base de polyester est un copolymère polyacide-L-lactique-poly-ε-caprolactone.

9. Matrice pour implant osseux selon la revendication 1 pour utilisation dans au moins un des domaines suivants : chirurgie osseuse reconstructive, chirurgie osseuse reconstructive maxillo-faciale, chirurgie orale, chirurgie dentaire, chirurgie orthopédique et implantologie.

10. Matrice pour implant osseux selon la revendication 1 pour utilisation en traitement humain ou vétérinaire.
